# EUROPEAN PATENT APPLICATION

(11) **EP 4 124 338 A1**
(43) Date of publication of application: **01.02.2023**
(21) Application number: 21306072.6
(22) Date of filing: 30.07.2021
(51) Int. Cl.: A61K 31/5375, A61P 3/10, A61P 27/00

(54) **SIGMA-1 RECEPTOR ACTIVATOR FOR USE IN THE TREATMENT OF A PATHOLOGY ASSOCIATED WITH WFS1 MUTATION**

(71) Applicant: Université de Montpellier, 34090 Montpellier (FR); Ecole Pratique des Hautes Etudes, 75014 Paris (FR); Institut National de la Santé et de la Recherche Médicale, 75013 Paris (FR)
(72) Inventor: DELPRAT, Benjamin, 34830 JACOU (FR); MAURICE, Tangui, 34980 SAINT-GÉLY-DU-FESC (FR)
(74) Representative: IPAZ

(57) **Abstract**

The present invention pertains to a compound for its use in the prevention or the treatment of a pathology associated with a mutation of the WFS1 gene. More particularly, the present invention concerns an activator of the Sigma-1 receptor (SIG-1R) for the prevention or the treatment of a pathology associated with a mutation of the WFS1 gene, in particular for the prevention or the treatment of Wolfram Syndrome and of Wolfram-like syndrome.

## Description

### FIELD OF THE INVENTION

The present invention pertains to a compound for its use in the prevention or the treatment of a pathology associated with a mutation of the WFS1 gene. More particularly, the present invention concerns an activator of the Sigma-1 receptor (SIG-1R) for the prevention or the treatment of a pathology associated with a mutation of the WFS1 gene, in particular for the prevention or the treatment of Wolfram Syndrome and of Wolfram-like syndrome.

### TECHNICAL BACKGROUND

The Wolfram syndrome (WS, OMIM # 222300, prevalence 1/180,000), is an emblematic form of inherited optic neuropathy (ION), because it associates to the progressive and rapid loss of vision leading to optical atrophy (OA), sensorineural hearing loss and various symptoms such as cerebellar ataxia and peripheral neuropathy. In addition to this wide neurosensory impairment, there is an early onset diabetes mellitus (DM) often associated to diabetes insipidus and hypogonadism. WS is an autosomal recessive genetic disease caused by mutations in WFS1 (HGNC: 12762 NCBI Entrez Gene: 7466 Ensembl: ENSG00000109501 OMIM^{®}: 606201 UniProtKB/Swiss-Prot: O76024).

It has recently been shown that recessive mutations in WFS1 also cause non syndromic forms of ION. In addition, beside WS and non-syndromic recessive ION, there are dominant forms of severe to profound hearing loss, which can be associated to OA and glucose intolerance, due to heterozygous mutations in WFS1, commonly denominated as WS-like diseases. Furthermore, WFS1 mutations are also responsible for rare cases of non-syndromic autosomal dominant diabetes, autosomal dominant diabetes and congenital hearing loss, or autosomal dominant congenital cataract. Moreover, decreased WFS1 expression is correlated with increased sensitivity to cisplatin. Indeed, the rs62283056 SNP localized in the first intron of WFS1 has been associated with increased hearing loss and decreased WFS1 expression by altering the binding motif of transcription factors such as E2F, HAND1, NANOG and POU5F1. Finally, a variant of WFS1, c.2500T>C, has been associated with a higher susceptibility to develop noise induced hearing loss, in a cohort of Taiwanese soldiers, frequently exposed to firearm blasts.

The "WFS1 gene" (gene bank ID: 7466) codes for wolframin, a predicted 890-amino acid transmembrane protein. It is located at position 4pl6.1 in the human genome and its mutation(s) is(are) responsible of the Wolfram Syndrome of type 1. The following mutations of WFS1, which are identified by their rs reference in the NCBI dbSNP Short Genetic Variations Database, are examples of mutations known to cause Wolfram Syndrome 1: rs28937890, rs28937891, rsl04893879, rs28937892, rsl04893880, rsl04893881, rs587776598, rs71524377.

WS symptoms were implemented together with clinical studies with other symptoms such as urinary disorders, pons atrophy and ataxia in about 50% of the patients. In addition, psychiatric disorders have been described, including bipolar disorder, schizophrenia, suicide or mania and were present in 39% of the patients. WS is a devastating pathology with unmet clinical needs, to date no treatment is available, except palliative treatment for diabetes, and patients die on average at 35 years old. Therefore, the need to find a cure is critical.

WFS1 gene encodes Wolframin (WFS1_HUMAN, UniProtKB: 076024 OMIM: 606201), an endoplasmic reticulum (ER) transmembrane protein involved in contacts between ER and mitochondria termed mitochondria associated-ER membranes (MAMs). Wolframin deficiency leads to an alteration of the communication between ER and mitochondria, triggering mitochondria dysfunction.

Mutations in WFS1 gene are not only associated with Wolfram Syndrome and Wolfram-like Syndrome, but also with symptoms such as: deafness and type II diabetes, even if Wolfram Syndrome as such is not present.

The Sigma receptor 1, also designated as SIG-1R (SIGMAR1 gene; HGNC: 8157 NCBI Entrez Gene: 10280 Ensembl: ENSG00000147955 OMIM^{®}: 601978 UniProtKB/Swiss-Prot: Q99720) is an intracellular protein mainly distributed, in mammals, on the endoplasmic reticulum membrane and particularly concentrated at the ER contacts with the mitochondria. The 223 amino acid sequence of human SIG-1R (NP_005857.1), and the nucleotide sequences encoding said protein (NM_005866.4) are publicly disclosed. It is known to have a chaperone-like function (T. Hayashi &T.P. Su, 2007). SIG-1R is involved in the transfer of calcium ions from the ER to the cytosol and mitochondrial, energy production, lipid synthesis and transport, and protein folding.

By stabilizing the conformation of inositol 1,2,4-trisphosphate receptor type 1 (IP3R1), SIG-1R enhances the efflux of Ca²⁺ from the ER into the cytosol and mitochondria (T. Hayashi *et al.,* 2000), thus improving mitochondria function. Indeed, SIG-1R agonists have a direct but dual effect on mitochondrial oxidative status depending on physiological or pathological conditions (N. Goguadze *et al.,* 2019).

US 2017360798A1 *"ANAVEX2-73 and certain anticholinesterase inhibitors composition and method for neuroprotection"* discloses that SIG-1R receptor activation is linked to neuroprotection against diseases such as Alzheimer's disease.

WO 2020/013108 discloses a pharmaceutical composition for preventing or treating mitochondrial diseases, wherein said composition contains a SIG-1R agonist. Mitochondrial diseases are defined as hereditary diseases that result in dysfunction of mitochondrial energy production and are caused by mutation of mitochondrial DNA or nuclear DNA encoding a protein involved in the mitochondrial electron transport system. It is now admitted that WS is not a mitochondrial disease, as it is not caused by a mutation of mitochondrial DNA or by a mutation of a gene encoding a protein involved in the mitochondrial electron transport system.

EP 2335688 « *Pharmaceutical compositions comprising sigma receptor ligands »,* discloses the use of ligands of SIG-1R for treating psychosis and diseases such as Huntington disease and Parkinson disease.

WO 2017/162798 discloses the targeting of the neuronal calcium sensor 1 for treating Wolfram Syndrome.

Targeting SIG-1R has not been disclosed for the prevention or the treatment of a pathology associated with a mutation of the WFS1 gene. More particularly, a SIG-1R activator was not considered for preventing or treating a pathology associated with a mutation of the WFS1 gene such as Wolfram Syndrome.

The inventors have now demonstrated that activation of SIG-1R could counteract the functional alterations due to wolframin deficiency. Overexpression of the SIG-1R protein, or treatment with the SIG-1R agonist PRE-084, was able to alleviate the behavioral symptoms observed in different genetic animal models of the disease, *i.e.* hyperlocomotion in *Wfs1ab^{KO}* zebrafish and memory deficits and anxiety in *Wfs1^{ΔExon8}* mice. This has been shown either by SIG-1R overexpression and by using the prototypic SIG-1R agonist, PRE-084.

Taken together, these data reveal SIG-1R as a pharmacological target useful for treating a pathology associated with a mutation of WFS1 gene. Increasing and/or stimulating the expression of SIG-1R should therefore be considered as a very efficient pathway for treating, among other pathologies, Wolfram Syndrome.

### SUMMARY OF THE INVENTION

In a first aspect, the present invention provides a compound for its use in the prevention or the treatment of a pathology associated with a mutation of WFS1, in particular a pathology chosen among the Wolfram syndrome and the Wolfram-like syndrome, wherein said compound is an activator of Sigma-1 receptor (SIG-1R).

In a second aspect, the present invention provides a pharmaceutical composition for its use in the prevention or the treatment of a pathology associated with a mutation of WFS1, said pharmaceutical composition comprising an activator of SIG-1R.

The present invention also provides a method of prevention or treatment of a pathology associated with a mutation of WFS1, said method comprising the administration, to a patient in need thereof, of an activator of SIG-1R.

Characteristics, advantages and uses of the subject-matter of the present invention are more detailed hereunder, in an illustrated and non-limiting way. When present, the disclosure of the ranges expressed as "from ... to ..." mean that the limits are included in said ranges.

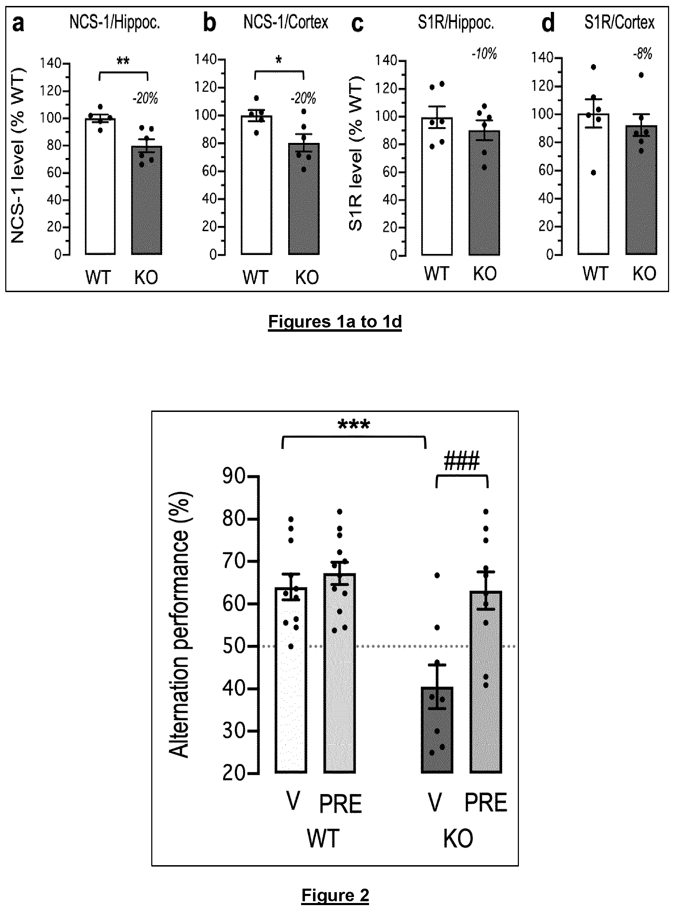

Figures 1a to 1d illustrates the altered expression of NCS-1 but not SIG-1R in Wfs^{Δexon8} mice. NCS-1 protein levels were decreased in the hippocampus (a) and cortex (b) of Wfs1^{WT} (WT) and Wfs1^{ΔExon8} (KO) mice. ^{∗} p < 0.05, ^{∗∗} p < 0.01 vs. WT mice; Newman-Keuls test. SIG-1R protein levels were unchanged in the hippocampus (c) and cortex (d) of WT and KO mice.

Figure 2 represents the SIG-1R agonist PRE-084 attenuating the deficits of spontaneous alternation observed in male Wfs1^{ΔExon8} mice. Vehicle solution (V) or PRE-084 (0.3 mg/kg ip) was administered in male Wfs1^{WT} (WT) and Wfs1^{ΔExon8} (KO) mice, 30 min before the Y-maze session. ^{∗∗∗} p < 0.001 vs. V-treated WT mice; ### p < 0.001 vs. V-treated KO mice; Newman-Keuls test.

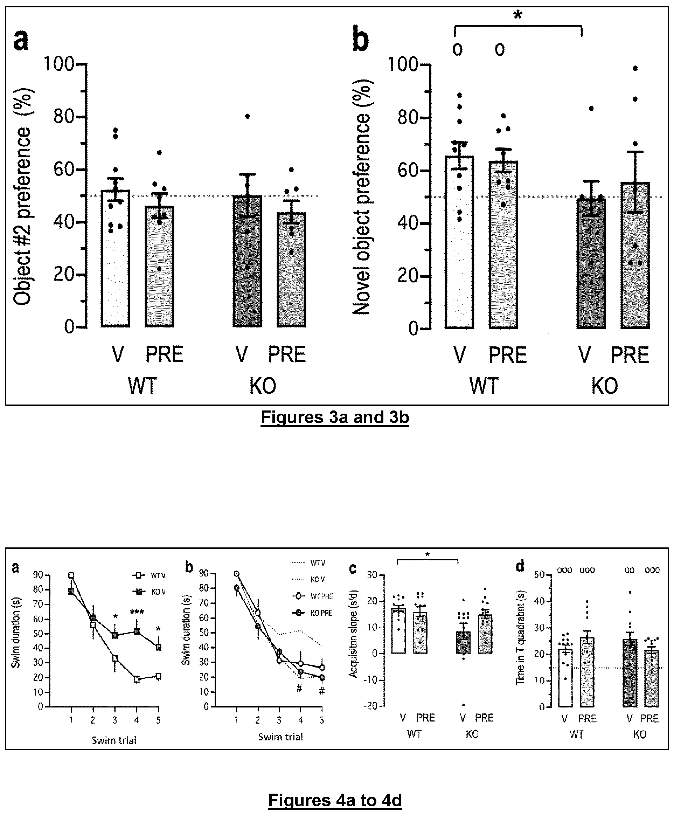

Figures 3a and 3b represent the SIG-1R agonist PRE-084 attenuating the recognition memory deficits in male Wfs1^{ΔExon8} mice. Vehicle solution (V) or PRE-084 (0.3 mg/kg ip) was administered in male Wfs1^{WT} (WT) and Wfs1^{ΔExon8} (KO) mice, 30 min before the session with two identical objects, shown in (b). The data from the session with a novel object is shown in (c). ^{∗} p < 0.05 vs. V-treated WT mice; Newman-Keuls test. ° p < 0.05 vs. 50% level, one-sample t-test.

Figures 4a to 4d represent the SIG-1R agonist PRE-084 attenuated acquisition deficits in the water-maze in male Wfs1^{ΔExon8} mice. Vehicle solution (V) or PRE-084 (0.3 mg/kg ip) was administered in male Wfs1^{WT} (WT) and Wfs1^{ΔExon8} (KO) mice, 30 min before the 1^{st} swimming in each training trial. Mice performed 3 swims per day to locate an immerged platform and the median latency was averaged each day. (a) V-treated WT and KO mice; (b) PRE-treated WT and KO mice. (c) Acquisition slopes were calculated individually for each treatment group. (d) Retention test: mice were allowed a 60-s swim in the water-maze without platform and presence in the training (T) quadrant was videotracked. ^{∗} p < 0.05, ^{∗∗∗} p < 0.001 vs. V-treated WT mice; # p < 0.05 vs. V-treated KO mice; Newman-Keuls test. °° p < 0.01, °°° p < 0.001 vs. 15-s level in (d), one-sample t-test.

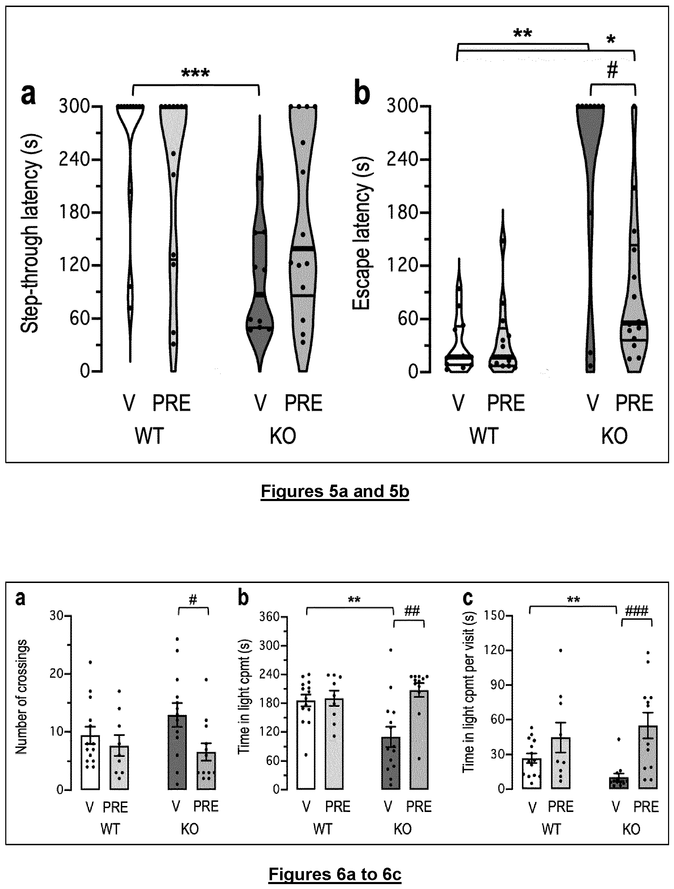

Figures 5a and 5b represent the SIG-1R agonist PRE-084 attenuating the passive avoidance deficits in male Wfs1^{ΔExon8} mice. Vehicle solution (V) or PRE-084 (0.3 mg/kg ip) was administered in male Wfs1^{WT} (WT) and Wfs1^{ΔExon8} (KO) mice, 30 min before the training session in the passive avoidance test, with step-through latency for retention shown in (a) and escape latency shown in (b). ^{∗} p < 0.05, ^{∗∗} p < 0.01, ^{∗∗∗} p < 0.001 vs. V-treated WT mice; # p < 0.05 vs. V-treated KO mice; Mann-Whitney's test.

Figures 6a to 6c show the SIG-1R agonist PRE-084 attenuating the deficits of anxiety in female Wfs1^{ΔExon8} mice tested in the light-and-dark exploration test. Vehicle solution (V) or PRE-084 (0.3 mg/kg ip) was administered in female Wfs1^{WT} (WT) and Wfs1^{ΔExon8} (KO) mice, 30 min before being placed in the light-and-dark box: (a) Number of crossings between the two compartments, (b) duration spent in the light compartment, and (c) time per visit. ^{∗∗} p < 0.01 vs. V-treated WT mice; # p < 0.05, ## p < 0.01, ### p < 0.001 vs. V-treated KO mice; Newman-Keuls test.

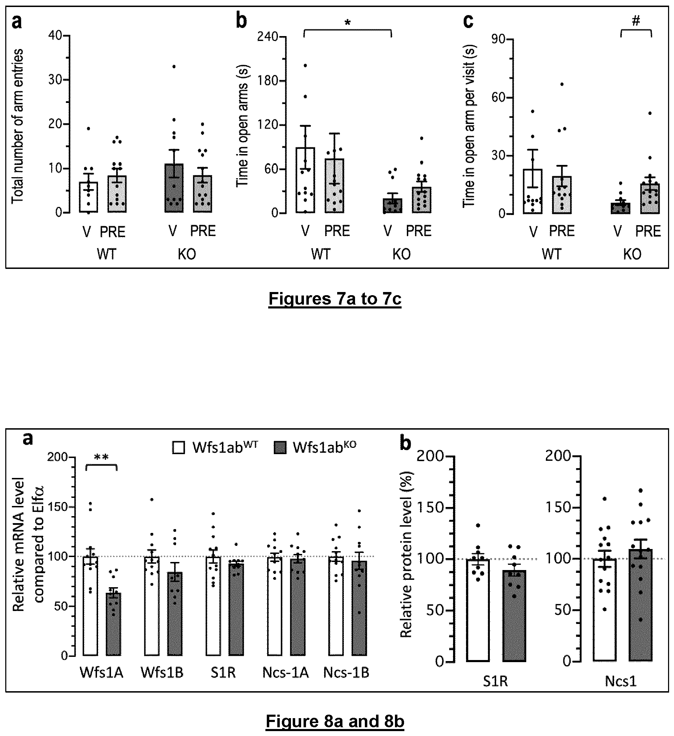

Figures 7a to 7c illustrate SIG-1R agonist PRE-084 attenuating the deficits of anxiety in female Wfs1^{ΔExon8} mice tested in the elevated plus-maze test. Vehicle solution (V) or PRE-084 (0.3 mg/kg ip) was administered in female Wfs1^{WT} (WT) and Wfs1^{ΔExon8} (KO) mice, 30 min before being placed in the elevated plus-maze: (a) total number of arm entries, (b) time in open arms, and (c) time per visit. ^{∗} p < 0.05 vs. V-treated WT mice; # p < 0.05 vs. V-treated KO mice; Newman-Keuls test.

Figure 8 shows the biochemical characterization of the Wfs1ab^{KO} zebrafish line. (a) Quantification of mRNA levels for Wfs1A, Wfs1B, SIG-1R, Ncs-1A, Ncs-1B in Wfs1ab^{WT} and Wfs1ab^{KO} larvae by RT-PCR. (b) Quantification of NCS-1 and SIG-1R proteins level. ^{∗∗} p < 0.01; Newman-Keuls test.

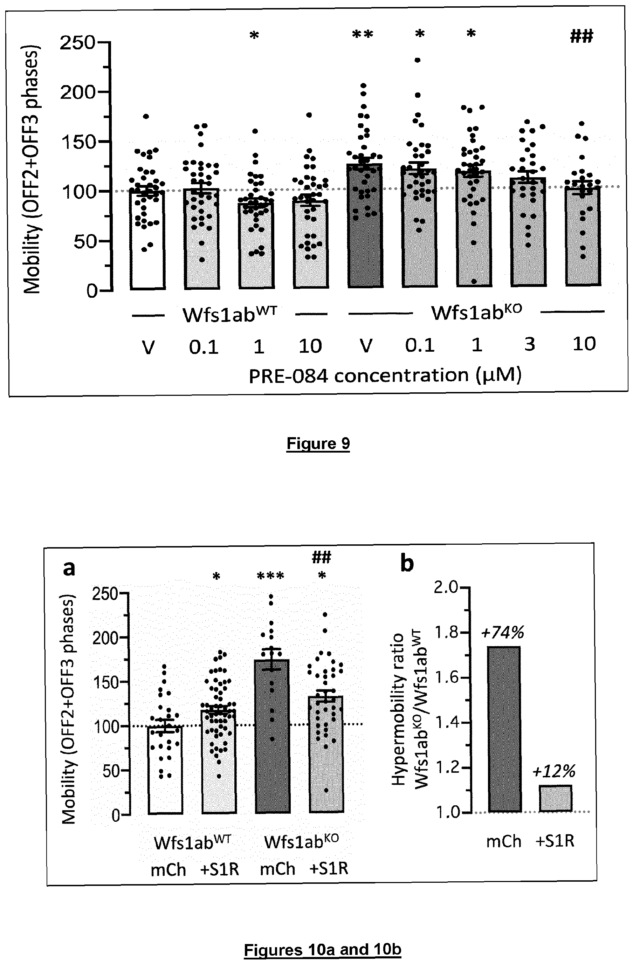

Figure 9 illustrates the dose-response effect of PRE-084 attenuating the hypermobility in Wfs1ab^{KO} zebrafish larvae. Quantification of cumulated mobility during the OFF2+OFF3 phases, expressed as % of control (V-treated Wfs1^{WT} larvae): dose-response effect of PRE-084 (0.1-10 µM). ^{∗} p < 0.05, ^{∗∗} p < 0.01 vs. control Wfs1ab^{WT}; ## p < 0.01 vs. Wfs1ab^{KO} larvae; Dunnett test.

Figures 10a and 10b show that over-expression of SIG-1R attenuated the hypermobility in Wfs1ab^{KO} zebrafish larvae. (a) Quantification of cumulated mobility during the OFF2+OFF3 phases, expressed as % of control (mCherry-treated Wfs1^{WT} larvae). (b) Hypermobility ratio. ^{∗} p < 0.05, ^{∗∗∗} p < 0.001 vs. control Wfs1ab^{WT}; ## p < 0.01 vs. Wfs1ab^{KO} larvae; Dunnett test.

### DETAILED DESCRIPTION OF THE INVENTION

In a first aspect, the present invention relates to a compound for its use in the prevention or the treatment of a pathology associated with a mutation of WFS1, wherein said compound is an activator of SIG-1R.

For the purpose of the invention, a "pathology associated with a mutation of WFS1" is defined as a condition, a symptom or pathology associated with at least one mutation in one of its WFS1 gene. Human WFS1 (HGNC: 12762 NCBI Entrez Gene: 7466 Ensembl: ENSG00000109501 OMIM^{®}: 606201 UniProtKB/Swiss-Prot: O76024) comprises 8 exons.

In a particular embodiment, the present invention relates to a compound for use in the prevention or the treatment of a pathology associated with a mutation of WFS1 wherein the Wolframin protein is deficient or is non-functional.

In the context of the invention, the term "treating" or "treatment", means reversing, alleviating, inhibiting the progress of, or preventing the disorder or condition to which such term applies, or one or more symptoms of such disorder or condition.

For the purpose of the invention, an activator of SIG-1R is defined as a compound able to increase at least one of the physiological functions of SIG-1R. In a particular embodiment, an activator of SIG-1R is a compound able to trigger the chaperone activity of SIG-1R, wherein said binding leads to an increase of at least one of the physiological functions of SIG-1R.

Physiological functions of SIG-1R comprise, but are not limited to, modulation of Ca²⁺ transfer among intracellular organelles (T. Hayashi *et al.,* 2000), dissociation from the endoplasmic reticulum (ER) stress sensor protein BiP/GRP78 and activation of the IRE-1-dependent and ATF6-dependent ER stress pathways (T. Hayashi & T.P. Su, 2007), potentiation of glutamatergic neurotransmission (F. Monnet *et al.,* 2012), potentiation of monaminergic neurotransmissions (Y. Minabe *et al.,* 1999), increased release of neurotrophic factors particularly brain-derived neurotrophic factor (BDNF) (Y. Yagasaki *et al.,* 2006), regulation of cellular excitability and PM channels (T.R. Anderson *et al.,* 2005) and cholesterol lipid scaffolding (V. Zhemkov *et al.,* 2021).

In a particular embodiment of this first aspect, the present invention relates to an activator of SIG-1R for its use in the prevention or the treatment of a pathology associated with a mutation of WFS1, wherein said pathology is a conditions or a disease chosen among the following: Wolfram syndrome, Wolfram-like syndrome, *diabetes insipidus, diabetes mellitus,* optic atrophy, deafness, psychiatric disorders, acoustic trauma and severity of hearing loss following cisplatin treatment.

In a more particular embodiment of this first aspect, the present invention relates to an activator of SIG-1R for its use in the prevention or the treatment of Wolfram syndrome.

The present invention relates in a more particular embodiment to an activator of SIG-1R for its use in the prevention or the treatment of a condition or a disease of a patient diagnosed with Wolfram syndrome or with Wolfram-like syndrome, for the prevention of the treatment of at least *diabetes insipidus, diabetes mellitus,* an optic atrophy and/or and deafness.

An activator of SIG-1R is a compound which, when it is added at an active dose in the appropriate testing conditions, leads to the observation of at least one of the following:
- potentiation of Ca²⁺ transfer from the endoplasmic reticulum, as shown in NG-108B cells (T. Hayashi *et al,* 2000), in a SIG-1R antagonist sensitive manner,
- increase of dissociation between BiP/GRP78 and Sig-1R, as shown in NG-108B cells (T. Hayashi & T.P. Su, 2007), in a SIG-1R antagonist sensitive manner,
- conformational changes of the chaperone protein using a receptor fluorescence resonance energy transfer (FRET) based biosensor containing CFP and YFP to monitor ligand-mediated conformational rearrangements by assessing real-time intramolecular FRET changes in HEK-293T cells (M. Gómez-Soler *et al.,* 2014),
- attenuation of Dizocilpine ((+)-MK-801 maleate)-induced learning impairment in rodents *in vivo* (T. Maurice *et al.,* 1994a, T. Maurice *et al.,* 1994b), in a SIG-1R antagonist sensitive manner.

More particularly, according to the present invention, an activator of SIG-1R is a compound characterized by its ability to induce at least one of the following:
- potentiation of Ca²⁺ transfer from the endoplasmic reticulum in NG-108B cells in a SIG-1R antagonist sensitive manner,
- increase of dissociation between BiP/GRP78 and SIG-1R, in NG-108B cells, in a SIG-1R antagonist sensitive manner.

In a particular embodiment of the first aspect of the present invention, an activator of SIG-1R is a ligand of SIG-1R chosen among SIG-1R agonists and SIG-1R positive modulators. The group of "SIG-1R positive modulators" comprises SIG-1R positive allosteric modulators (PAM). SIG-1R ligands are for example disclosed in (T. Maurice, 2021).

For the purpose of the invention, an activator of SIG-1R which is an agonist of SIG-1R is defined as a compound able to bind to the SIG-1R binding site labeled with the radiotracer [³H](+)-pentazocine ([³H](2R,6R,11R)-1,13-dimethyl-10-(3-methylbut-2-en-1-yl)-10-azatricyclo[7.3.1.02,7]trideca-2,4,6-trien-4-ol), for instance, with a Ki comprised between 1 µM and 0.01 nM. In a particular embodiment of the invention, an agonist of SIG-1R binds to SIG-1R with a selectivity vs. sigma-2 receptors of at least 100 × (Ki(SIG-1R) < Ki(SIG-2R) / 100).

Known agonists of SIG-1R include but are not limited to the compounds such as: (+)-3-PPP ((+)-3-(1-Propylpiperidin-3-yl)phenol); (+)-N-Alazocine ([2S-(2a, 6a,11R)]-1,2,3,4,5,6-Hexahydro-6,11-dimethyl-3-(2-propenyl)-2,6-methano-3-benzazocin-8-ol hydrochloride, (+)-SKF-10047, allylnormetazocine); (+)-Pentazocine ((2R,6R,11R)-1,13-dimethyl-10-(3-methylbut-2-en-1-yl)-10-azatricyclo[7.3.1.02,7]trideca-2,4,6-trien-4-ol); 4-IBP (N-(N-benzylpiperidin-4-yl)-4-iodobenzamide); 4-PPBP (4-Phenyl-1-(4-phenylbutyl)piperidine); AF-701B (1-(2,8-Dimethyl-1-thia-3,8-diazaspiro(4.5)dec-3-yl)-3-(1H-indol-3-yl)propan-1-one; ANAVEX3-71); ANAVEX1-41 (tetrahydro-N,N-dimethyl-4,4-diphenyl-3-furanmethanamine hydrochloride); Arketamine ((R)-2-(2-Chlorophenyl)-2-(methylamino)cyclohexanone, (R)-ketamine); BD737 (cis-N-(2-(3,4-Dichlorophenyl)ethyl)-N-methyl-2-(1-pyrrolidinyl)cyclohexylamine); BD1031 ((8aR)-2-[2-(3,4-dichlorophenyl)ethyl]octahydropyrrolo[1,2-a]pyrazine); BD1052 (N-[2-(3,4-dichlorophenyl)ethyl]-N-(2-pyrrolidin-1-ylethyl)prop-2-en-1-amine); Berberine (16,17-dimethoxy-5,7-dioxa-13-azoniapentacyclo[11.8.0.0^{2,10}.0^{4,8}.0^{15,20}]henicosa-1(13),2,4(8),9,14,16,18,20-octaene); Blarcamesine (tetrahydro-N,N-dimethyl-2,2-diphenyl-3-furanmethanamine hydrochloride; ANAVEX2-73); Carbetapentane (2-[2-(diethylamino)ethoxy]ethyl 1-phenylcyclopentanecarboxylate, Pentoxyverine); Cutamesine (1-[2-(3,4-dimethoxyphenyl)ethyl]-4-(3-phenylpropyl)piperazine; SA4503); Dehydroepiandrosterone (DHEA); Dehydroepiandrosterone sulfate (DHEA-S); Dextromethorphan ((1*S*,9*S*,10*S*)-4-methoxy-17-methyl-17-azatetracyclo[7.5.3.0^{1,10}.0^{2,7}]heptadeca-2(7),3,5-triene); Dextrorphan ((1S,9S,10S)-17-methyl-17-azatetracyclo[7.5.3.0^{1,10}.0^{2,7}]heptadeca-2(7),3,5-trien-4-ol); Dimemorfan ((1*S*,9*S*,10*S*)-4,17-dimethyl-17-azatetracyclo[7.5.3.0^{1,10}.0^{2,7}]heptadeca-2(7),3,5-triene); Ditolylguanidine (1,2-bis(2-methylphenyl)guanidine); Edonerpic maleate (1-{3-[2-(1-benzothiophen-5-yl)ethoxy] propyl}-3-azetidinol , T-817MA); Fabomotizole (4-[2-[(6-ethoxy-1*H*-benzimidazol-2-yl)sulfanyl]ethyl]morpholine); Fluoxetine (N-methyl-3-phenyl-3-[4-(trifluoromethyl)phenoxy]propan-1-amine); Fluvoxamine (2-{[(E)-{5-methoxy-1-[4-(trifluoromethyl)phenyl]pentylidene}amino]oxy}ethanamine); Igmesine ((+)-cinnamyl-1-phenyl-1-N-methyl-N-cyclopropylene); L-687,384 (1'-benzylspiro[2,3-dihydro-1H-naphthalene-4,4'-piperidine]); Lamotrigine (6-(2,3-dichlorophenyl)-1,2,4-triazine-3,5-diamine); Methylphenidate (methyl 2-phenyl-2-piperidin-2-ylacetate); (-)-MR22 ((-)-methyl (1S,2R)-2-{[1-adamantyl(methyl)amino]methyl}-1-phenylcyclopropane-carboxylate)); N,N-Dimethyltryptamine; Noscapine ((3S)-6,7-dimethoxy-3-[(5*R*)-4-methoxy-6-methyl-7,8-dihydro-5*H*-[1,3]dioxolo[4,5-g]isoquinolin-5-yl]-3*H*-2-benzofuran-1-one); OPC-14523 (1-(3-(4-(3-chlorophenyl)-1-piperazinyl)propyl)-3,4-dihydro-5-methoxy-2(1H)-quinolinone); Opipramol (2-[4-(3-benzo[b][1]benzazepin-11-ylpropyl)piperazin-1-yl]ethanol); PPCC ((S^{∗},R^{∗})-2-[(4-hydroxy-4-phenyl-1-piperidinyl)methyl]-1-(4-methylphenyl)-cyclopropanecarboxylic acid methyl ester); PRE-084 (2-morpholin-4-ylethyl-1-phenylcyclohexane-1-carboxylate); Pregnenolone; Pregnenolone sulfate; Pridopidine (4-(3-(methylsulfonyl)phenyl)-1-propylpiperidine); RC-33 (1-[1-(4-biphenyl)-1-methylpropyl]piperidine); Sertraline ((1S,4S)-4-(3,4-dichlorophenyl)-N-methyl-1,2,3,4-tetrahydronaphthalen-1-amine); Siramesine (1'-{4-[1-(4-Fluorophenyl)-1H-indol-3-yl]butyl}-3H-spiro[2-benzofuran-1,4'-piperidine], Lu-28-179).

By "allosteric modulator" it is intended a compound that modulates SIG-1R activity by binding SIG-1R at a different than the orthosteric site (or active site) of SIG-1R. The binding of an allosteric modulator to SIG-1R induces a conformational change in the protein structure and changes the activity of orthosteric ligands.

According to the invention, an activator of SIG-1R is chosen among the groups consisting of: selective active modulators of SIG-1R and non-selective active modulators of SIG-1R, such as defined by (E. Vavers *et al.,* 2019), wherein active modulators of SIG-1R include but are not limited to: Phenytoin (5,5-diphenylimidazolidine-2,4-dione); SKF-83959 (6-chloro-7,8-dihydroxy-3-methyl-1-(3-methylphenyl)-2,3,4,5-tetrahydro-1H-3-benzazepine); methylphenylpiracetam (2-(5-methyl-2-oxo-4-phenyl-pyrrolidin-1-yl)-acetamide, E1R); OZP002 (±)-2-(3-chlorophenyl)-3,3,5,5-tetramethyl-2-oxo-[1,4,2]-oxazaphosphinane); Ropizine ((E)-N-(4-benzhydrylpiperazin-1-yl)-1-(6-methylpyridin-2-yl)methanimine); SKF-38393 ((±)-1-phenyl-2,3,4,5-tetrahydro-(1H)-3-benzazepine-7,8-diol hydrobromide); SCH-23390 (7-chloro-3-methyl-1-phenyl-1,2,4,5-tetrahydro-3-benzazepin-8-ol); SOMCL-668 (3-methyl-phenyl-2, 3, 4, 5-tetrahydro-1H-benzo[d]azepin-7-ol); fenfluramine ((RS)-N-ethyl-1-[3-(trifluoromethyl)phenyl]propan-2-amine).

More particularly, the present invention relates to an activator of SIG-1R for its use in the prevention or the treatment of a pathology associated with a mutation of WFS1, wherein said activator of SIG-1R is chosen among the groups of: molecules, peptides and steroids.

Activators of SIG-1R include, in particular, steroids: pregnenolone, pregnenolone sulfate, dehydroepiandrosterone, dehydroepiandrosterone sulfate.

Among peptides, antibodies, antibodies fragments and monoclonal antibodies can be cited for example. Among molecules, chemical druggable molecules can be cited for example.

More particularly, the present invention relates to an activator of SIG-1R for its use in the prevention or the treatment of a pathology associated with a mutation of WFS1, wherein said activator of SIG-1R is 2-(4-morpholinethyl)-1-phenylcyclohexanecarboxylate hydrochloride (PRE-084).

In another particular embodiment of the first aspect of the present invention, an activator of SIG-1R is the SIG-1R protein itself or a fragment thereof. A fragment of SIG-1R is a protein which amino acid sequence corresponds to at least 50 amino acids of the amino acid sequence of SIG-1R, preferably at least 70, 80 or 100 amino acids of the amino acid sequence of SIG-1R. In a particular embodiment, a fragment of SIG-1R is a protein which amino acid sequence corresponds to amino acids 116 to 223 of the amino acid sequence of SIG-1R. In another particular embodiment, a fragment of SIG-1R is a protein which amino acid sequence corresponds to amino acids 102 to 223 of the amino acid sequence of SIG-1R.

Overexpression of the SIG-1R, or of an active fragment thereof, has been shown to trigger its chaperone activity. (T. Hayashi & T.P. Su, 2007) showed that the SIG-1R fragment [amino acids 116-223] carried the chaperone activity of the whole SIG-1R protein in the light scattering assay. (Z. Wu and W.D. Bowen, 2008) showed that the overexpression of the whole SIG-1R protein [amino acids 1-223] or a C-terminal fragment [amino acids 102-223] drove a ligand-independent activation. The present disclosure shows (Figures 10a and 10b) that direct overexpression of the whole SIG-1R protein prevented the hyperlocomotor response of Wfs1ab^{KO} zebrafish larvae similarly as seen with the reference SIG-1R agonist PRE-084.

In another particular embodiment of this first aspect of the present invention, an activator of SIG-1R is the SIG-1R protein, or a fragment thereof. In this embodiment of the present invention, SIG-1R, or a fragment thereof, may be administered as a protein, or fragment thereof, or as a nucleotide sequence encoding for said protein or fragment thereof.

The present invention therefore also relates to a polynucleotide encoding for SIG-1R or for a fragment thereof, for its use in the prevention or treatment of a pathology associated with a mutation of WFS1, wherein said compound is an activator of SIG-1R. The present invention also provides a method for treating a pathology associated with a mutation of WFS1, comprising administering, to a patient in need thereof, a polynucleotide encoding for SIG-1R or for a fragment thereof.

Production of a suitable gene product may be achieved using recombinant techniques. For example, a suitable vector may be inserted into a host cell and expressed in that cell. Thus, the invention relates to a method for treating a pathology associated with a mutation of WFS1, which comprises the step of administering in a subject in need thereof a nucleic acid sequence that encodes a functional SIG-1R or a fragment thereof, so that it is expressed *in vivo* by the cells of the subject that have been transfected with said polynucleotide. The invention also relates to the use of a SIG-1R-encoding polynucleotide for the manufacture of a medicament intended for the treatment of a pathology associated with a mutation of WFS1. Said SIG-1R-encoding polynucleotide is administered in a therapeutically effective amount.

Preferably the SIG-1R sequence according to the invention is associated with elements that enable for regulation of its expression, such as a promoter sequence.

Such a nucleic acid may be in the form of a vector. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors including, but not limited to, replication defective retroviruses, adenoviruses, lentiviruses and adeno-associated viruses (AAV), which serve equivalent functions.

Different types of AAV vectors can be used depending on the type of administration and on the tissue to be targeted. The SIG-1R-encoding polynucleotide may be introduced into a target cell by means of any procedure known for the delivery of nucleic acids to the nucleus of cells, *ex vivo,* on cells in culture or removed from an animal or a patient, or *in vivo.*

*Ex vivo* introduction may be performed by any standard method well known by one skilled in the art, including, but not limited to, transfection, electroporation, microinjection, transduction, cell fusion, DEAE dextran, calcium phosphate precipitation, or use of a gene gun.

The SIG-1R-encoding polynucleotide can also be introduced *ex vivo* or *in vivo* by lipofection. In certain embodiments, the use of liposomes and/or nanoparticles is contemplated for the introduction of the donor nucleic acid targeting system into host cells. Nanocapsules can generally entrap compounds in a stable and reproducible way. Liposomes are formed from phospholipids that are dispersed in an aqueous medium and spontaneously form multilamellar concentric bilayer vesicles, also termed multilamellar vesicles (MLVs). MLVs generally have diameters of from 25 nm. Sonication of MLVs results in the formation of small unilamellar vesicles (SUVs) with diameters in the range of 200 to 500 A, containing an aqueous solution in the core.

Synthetic cationic lipids designed to limit the difficulties and dangers encountered with liposome mediated transfection can be used to prepare liposomes for *in vivo* transfection of a gene encoding a marker.

The SIG-1R -encoding polynucleotide across cell membranes *in vivo* may involve the direct application of high concentration free or naked polynucleotides (typically mRNA or DNA). By "naked DNA (or RNA)" is meant a DNA (RNA) molecule which has not been previously complexed with other chemical moieties. Naked DNA uptake by animal cells may be increased by administering the cells simultaneously with excipients and the nucleic acid. Such excipients are reagents that enhance or increase penetration of the DNA across cellular membranes and thus delivery to the cells delivery of the therapeutic agent. Various excipients have been described in the art, such as surfactants, e.g. a surfactant selected form the group consisting of Triton X-100, sodium dodecyl sulfate, Tween 20, and Tween 80; bacterial toxins, for instance streptolysin O, cholera toxin, and recombinant modified labile toxin of E coli; and polysaccharides, such as glucose, sucrose, fructose, or maltose, for instance, which act by disrupting the osmotic pressure in the vicinity of the cell membrane. Other methods have been described to enhance delivery of free polynucleotides, such as blocking of polynucleotide inactivation via endo- or exonucleolytic cleavage by both extra- and intracellular nucleases.

Knowing the sequence of the SIGMAR1 gene (HGNC: 8157 NCBI Entrez Gene: 10280 Ensembl: ENSG00000147955 OMIM^{®}: 601978 UniProtKB/Swiss-Prot: Q99720), one skilled in the art can readily produce said polypeptides, by standard techniques for production of polypeptides. For instance, they can be synthesized using well-known solid phase method, preferably using a commercially available peptide synthesis apparatus (such as that made by Applied Biosystems, Foster City, California) and following the manufacturer's instructions.

Alternatively, the polypeptides of the invention can be synthesized by recombinant DNA techniques as is now well-known in the art. For example, these fragments can be obtained as DNA expression products after incorporation of DNA sequences encoding the desired (polypeptide into expression vectors and introduction of such vectors into suitable eukaryotic or prokaryotic hosts that will express the desired polypeptide, from which they can be later isolated using well-known techniques. Polypeptides of the invention can be use in an isolated (e.g., purified) form or contained in a vector, such as a membrane or lipid vesicle (e.g. a liposome).

In a second aspect, the present invention relates to a pharmaceutical composition comprising, as an active ingredient, an activator of SIG-1R or a pharmaceutically acceptable salt thereof, for its use in the prevention or the treatment of a pathology associated with a mutation of WFS1.

A pharmaceutical composition according to the present invention comprises a therapeutically effective amount of an activator of SIG-1R. A "therapeutically effective amount" is intended for a minimal amount of active agent which is necessary to impart therapeutic benefit to a subject. For example, a "therapeutically effective amount" to a mammal is such an amount which induces, ameliorates or otherwise causes an improvement in the pathological symptoms, disease progression or physiological conditions associated with or resistance to succumbing to a disorder.

More particularly, the present invention relates to a pharmaceutical composition comprising, as an active ingredient, at least one activator of SIG-1R or a pharmaceutically acceptable salt thereof.

The pharmaceutical composition of the present invention may consist essentially of the active ingredient and at least one physiologically compatible carrier, excipient or diluent.

In a particular embodiment, the present invention relates to a pharmaceutical composition comprising an activator of SIG-1R or a pharmaceutically acceptable salt thereof, for its use in the prevention or the treatment of Wolfram syndrome or of Wolfram like syndrome.

In a more particular embodiment, the present invention relates to a pharmaceutical composition for its use in the prevention or the treatment of a pathology associated with a mutation of WFS1, said pharmaceutical composition comprising an agonist of SIG-1R.

Examples of dosage forms used for administration of the pharmaceutical composition of the present invention include tablets, granules, powders, capsules, syrups, suspensions, injections, and eye drops. Agents, ointments, patches and the like. These dosage forms can be manufactured using a technique widely used as a usual preparation method, for example, tablets, capsules, oral preparations such as granules, lactose, starch, crystalline cellulose as needed, vegetable oils and other bulking agents, magnesium stearate, lubricants such as talc, binders such as hydroxypropylcellulose and polyvinylpyrrolidone, disintegrators such as calcium carboxymethylcellulose, coating agents such as hydroxypropylcellulose, macrogol and silicone resin The active ingredient can be formulated using a gelatin coating agent.

For example, a pharmaceutical composition of the invention comprises 0.1 mg, 10 mg, 20 mg, 50 mg, or 100 mg or the like as a dosage unit amount of the active ingredient.

The present invention also provides a method of prevention or treatment of a pathology associated with a mutation of WFS1, said method comprising the administration, to a patient in need thereof, of pharmaceutical composition comprising an activator of SIG-1R.

For a method according to the present invention, said administration is for example chosen among oral, parenteral, intravenous, intramuscular, transdermal, eye drops, intra-auricular or subcutaneous. These administration methods and dosage forms are to be selected according to the patient's condition, age and treatment.

The present invention is particularly for mammalian, preferably humans.

### EXAMPLES

### Example 1: Neurological and psychiatric alterations of Wfs1^{ΔExon8} mice

### 1.1. Materials and methods

***Mouse breeding:** Wfs1^{ΔExon8}* founder mice were provided by Dr Sulev Koks (University of Tartu, Estonia). Generation of *Wfs1^{ΔExon8}* mice harboring β-galactosidase transgene were generated using a Wfs1 targeting construct deleting more than 90% of exon 8 and 60% of the total coding sequence including 8 of the 9 predicted transmembrane domains (Luuk, 2008). The construct was electroporated into W4/129S6 embryonic stem (ES) cells which were selected for resistance to Neomycin and Gancyclovir and one ES cell clone was injected into C57BL/6 blastocysts to establish heterozygous F1 mice by mating male chimeras with C57BL/6 female mice. F2 generation homozygous *Wfs1^{ΔExon8}* animals were obtained by crossing heterozygous F1 mice. Mice were genotyped by multiplex PCR for both alleles using primers WfsKO_wtF2 5'-TTGGCTTGTATTTGTCGGCC, NeoR1 5'-GACCGCTATCAGGACATAGCG and WfsKO_uniR2 5'-CCCATCCTGCTCTCTGAACC. The colony was propagated by heterozygous breeding (at least 5 generations) in the animal facility of the Neuroscience Institute of Montpellier. 2.5 months-old animals were transferred to the animal facility of the University of Montpellier (CECEMA) for experiments. Animals were housed in group, allowed food and water *ad libitum* except during experiments. They were maintained in a controlled environment (22 ± 1°C, 55 ± 5% humidity) with a 12 h:12 h light/dark cycle, lights on at 7:00 h. Experiments have been performed in 3-4 months old littermates. Female and male 129S6/SvEvTacxC57BL/6 mice (WT) and female and male *Wfs1^{ΔExon8}* mice (KO) were used in this study. Behavioral testing was performed between 10:00 and 16:00 h. All animal procedures were conducted in strict adherence to the European Union Directive 2010/63 and the ARRIVE guidelines and authorized by the National Ethic Committee (Paris) (APAFIS # 2018051411079082 #15035).

***Drugs and administration procedures:*** 2-(4-morpholinethyl)-1-phenylcyclohexanecarboxylate hydrochloride (PRE-084) was from Sigma-Aldrich (Saint-Quentin-Fallavier, France). It was solubilised in physiological saline (vehicle solution) and administered intraperitoneally (IP) at the dose of 1 mg/Kg, in a volume of 5 ml/Kg body weight, 30 min before the behavioural tests.

***Behavioral studies, open-field behavior:*** The general mobility of mice was first examined using an open-field procedure. Two concentric circles (Ø 15 cm and 45 cm) were drawn on the floor of a circular wooden arena (Ø 75 cm), with the outer ring being divided into 8 partitions and the middle ring into 4 partitions. The open-field session consisted of placing the mouse in the center circle and monitoring its movements for 10 min using a video camera. The following parameters were evaluated manually *ex tempora* by an experienced experimenter: (1) the time taken to move out of the center circle; (2) locomotion activity, in terms of distance traveled (m) calculated from the number of partitions crossed; (3) immobility duration; (4) locomotion speed, calculated as total distance over time in movement (total session time minus immobility and latency to start the exploration); (5) locomotion activity in the five central partitions; (6) number of rearing behaviors; (7) number of grooming behaviors; and (8) number of defecations. Animals that failed to move more than 1.5 m in 10 min were discarded from the calculations. Four mice were discarded accordingly corresponding to 8% attrition.

***Spontaneous alternation in the Y-maze:*** The test assesses spatial working memory. The maze was made of grey polyvinylchloride. Each arm was 40 cm long, 13 cm high, 3 cm wide at the bottom, 10 cm wide at the top, and converging at an equal angle. Each mouse was placed at the end of one arm and allowed to move freely through the maze during an 8 min session. The series of arm entries, including possible returns into the same arm, was recorded visually. An alternation was defined as entries into all three arms on consecutive occasions. The number of maximum alternations was therefore the total number of arm entries minus two and the percentage of alternation was calculated as (actual alternations/maximum alternations) × 100. Animals that failed to explore more than 8 arms in 8 minutes were discarded from the calculations. Thirteen animals were discarded accordingly corresponding to 26% attrition.

***Step-through type passive avoidance test:*** The test measures non-spatial long-term memory. The apparatus is a two-compartments (15 × 20 × 15 cm high) box with one illuminated with white polyvinylchloride walls and the other darkened with black polyvinylchloride walls and a grid floor. A guillotine door separates each compartment. A 60 W lamp positioned 40 cm above the apparatus lights up the white compartment during the experiment. Scrambled foot shocks (0.3 mA for 3 s) could be delivered to the grid floor using a shock generator scrambler (Lafayette Instruments, Lafayette, USA). The guillotine door was initially closed during the training session. Each mouse was placed into the white compartment. After 5 s, the door was raised. When the mouse entered the darkened compartment and placed all its paws on the grid floor, the door was closed and the foot shocks delivered for 3 s. The step-through latency, that is, the latency spent to enter the darkened compartment, and the shock sensitivity (estimated as 0 = no reaction, 1 = flinching reactions, 2 = flinchings and vocalizations) were recorded. The retention test was performed 24 h after training. Each mouse was placed again into the white compartment. After 5 s, the door was raised. The step-through latency and escape latency (corresponding to the time spent to exit the dark compartment) were recorded up to 300 s each. No exclusion criterion has been set in this test.

***Object recognition test:*** The test measures short-term recognition memory. The apparatus consisted in four squared open-fields (50 cm × 50 cm × 50 cm high) made in white Plexiglas and placed on a floor equipped with infrared (IR) light emitting diodes. The locomotor activity of the animal and position of their nose could be captured through an IR-sensitive camera and analyzed using the Videotrack^{®} and Nosetrack^{®} softwares (Viewpoint). Session 1: animals were allowed to acclimate during 10 min to the open-field. The mouse locomotion was analyzed by videotracking. Session 2: two identical objects (50 ml plastic vials with caps) were placed at defined positions, at ¼ and ¾ of one diagonal of the arena. Each mouse was placed in the open-field and the exploratory activity and nose position was recorded during 10 min. The activity was analyzed by videotracking in terms of number of contacts with the objects and duration of contacts. Session 3: the object in position 2 was replaced by a novel one (a soft plastic chair feet protection) differing in color shape and texture from the familiar object. Each mouse was placed again in the open-field and the exploratory activity recorded during 10 min. The activity was analyzed similarly. The preferential exploration index was calculated as the ratio of the number of contacts with the object in position 2 over the total number of contacts with the two objects. Animals showing no contact with one of the objects during the session 2 or 3 were discarded from the calculations. Thirty-two animals were discarded accordingly, corresponding to 33% attrition.

***Place learning in the water-maze:*** Spatial reference memory was analyzed using a water-maze procedure. A transparent Plexiglas non-slippery platform (Ø 10 cm) was immersed in a circular pool (Ø 140 cm, height 40 cm), under the water surface during acquisition. The water temperature, 23 ± 1°C, light intensity, external cues in the room, and water opacity were rigorously reproduced. Swimming was videotracked (Viewpoint), with trajectories being analyzed as latencies and distances. The software divided the pool into four quadrants. Training consisted in three swims per day for 6 days, with 15 min intertrial-time interval. Start positions, set at each limit between quadrants, were randomly selected and each mouse was allowed a 90 s swim to find the platform, initially placed in the NE quadrant. If at the end of the 90 s the animal did not find the platform, it was placed and left for 20 s on it. The median latency and swim path length were calculated for each training day. Acquisition slopes were also calculated individually over the acquisition period. Forty-eight hours after the last swim, on day 7, a probe test was performed during which the platform was removed and each animal allowed a free 60 s swim. The time spent in the training (T) quadrant and the average of time spent in the 3 others (o) were determined. On days 8, mice were re-tested in the water-maze for assessing visual ability and non-spatial learning. They were submitted to the acquisition of a visible platform placed in a new location, with training consisting in three swims per day with 15 min intertrial-time interval. Swimming was analyzed as latencies. No exclusion criterion has been set in this test.

***Black-and-white exploration box test:*** Anxiety level of WFS1 KO mice was first examined using the black-and-white exploration box. It consisted of two adjacent compartments (23 × 16 × 10 cm³) separated by a small opening (6 × 6 cm²). The light compartment is brightly lit from above and covered with a transparent lid, and the dark compartment is covered with a black lid. Mice were placed in the light box, and exploration measured during 10 min by IR videotracking (Viewpoint). The time spent in each compartment and number of crossings among compartments were recorded. The time in the light compartment per visit was calculated. Animals that did not enter the dark compartment were excluded. Seven animals were discarded accordingly corresponding to 14% attrition.

***Elevated plus-maze test:*** The apparatus consisted of two open arms (24 × 8 cm²), in white PVC, and two enclosed arms (24 × 8 × 20 cm³), in dark grey PVC. The arms extended from a central platform (8 × 8 cm), and the maze was elevated to a height of 50 cm above the floor. Each mouse was placed at the centre of the plus-maze and its exploration behaviour was recorded during 10 min. The time spent and numbers of entries into the enclosed or open arms were recorded. Results were expressed as total number of entries into the arms, time spent in the open arms, time spent per visit in the open arms.

***Experimental series:*** For phenotyping, animals were tested in series in the different behavioral tests assessing activity, learning and memory or anxiety, finishing with the most stressful procedures: (1) open-field (1 day), (2) spontaneous alternation (1 day), (3) object recognition (2 days), (4) place learning in the water-maze water-maze (5+2 days), (5) passive avoidance (2 days), (6) elevated plus-maze (1 day), and (7) black-and-white box exploration (1 day), in that order and over 16 days. For PRE-084 treatment experiments, animals were tested in series on a limited number of tests: spontaneous alternation (1 day), object recognition (2 days) and passive avoidance (2 days) for males, elevated plus-maze (1 day) and black-and-white box (1 day) for females.

### 1.2. Results

The mouse model of WS with complete knockout of exon 8, the *Wfs1^{ΔExon8}* line, provided by Dr S. Koks (Maurice, 2020), resulted in the deletion of amino acids 360-890 in the Wfs1 protein and the fusion between Wfs1 residues 1-360 and LacZ. To date more than 170 mutations have been identified in WFS1 with all types of mutations present (nonsense, missense, frameshift). The deletion of murine exon 8 should be similar to the human mutation Q366X of WFS1.

Behavioral phenotyping of the mouse line showed marked behavioral alterations. First, locomotor alterations were observed depending on the open-field paradigm (circular *vs.* squared arena for instance), but they convergently showed that *Wfs1^{ΔExon8}* mice presented a hypolocomotor response. Second, *Wfs1^{ΔExon8}* mice presented memory deficits that were very consistent in males and observable in all the procedures tested. Deficits were particularly marked for spontaneous alternation and novel object tests, evaluating an episodic-like form of recognition memory. The passive avoidance retention parameters that assessed contextual long-term memory, were also altered as well as acquisition of place learning in the water maze. Third, anxiety-related responses, measured using the presence in the center area of open-field arenas, the time spent in open arms in the elevated plus-maze, or in the enlighten compartment of the black-and-white exploration box, were also altered in *Wfs1^{ΔExon8}* mice and particularly in a consistent way in female mice.

### Example 2: SIG-1R is a target for a pharmacological intervention in Wfs1^{ΔExon8} mice

### 2.1. Materials and methods

**Zebrafish Generation:** All experiments were performed in accordance with the 2010/63/EU Directive and the ARRIVE guidelines. Zebrafish were purchased at the KIT - European Zebrafish Resource Center. Heterozygous *Wfs1a^{C799X}* and *Wfs1b^{W493X}* mutant line were maintained in an automated fish tank system (ZebTEC, Tecniplast) at 28°C, pH 7, conductivity around 500 µS and with a 14h:10h light:dark cycle. They were fed with standard diet. Heterozygous *Wfs1a*^{*C799X*/+} zebrafish were crossed to generate wild-type (*Wfs1a^{WT}*) and homozygous mutant *Wfs1a^{C799X}* zebrafish (*Wfs1a^{C799X}*), which became progenitors in order to generate future generations for the experiments. Heterozygous *Wfs1b*^{*W493X*/+} zebrafish were crossed to generate wild-type (*Wfs1b^{WT}*) and homozygous mutant *Wfs1b^{W493X}* zebrafish (*Wfs1b^{W493X}*), which became progenitors in order to generate future generations for the experiments. *Wfs1a^{C799X}* and *Wfs1b^{W493X}* were crossed to obtain *Wfs1ab^{WT}* and *Wfs1ab^{KO}.* Embryos were collected from natural breeding, raised at 28.5°C in E3 medium (5 mM NaCl, 0.17 mM KCI, 0.33 mM CaCl2, 0.33 mM MgSO4, 0.05% methylene blue). Unfertilized embryos were discarded and the water was changed only the first day.

***mRNA injection:*** Transcripts encoding human wild-type SIG-1R were generated after insertion of SIG-1R coding sequence in the pCS2+ plasmid. The cDNA encoding human wild-type SIG-1R was initially inserted in pCI-neo vector. After digestion by EcoR1 and XhoI restriction enzymes, the purified SIG-1R encoding nucleotide fragment was inserted between the EcoR1 and XhoI sites of the pCS2+ plasmid. SIG-1R mRNAs were transcribed from Not1-linearized pCS2+ using the SP6 polymerase with the mMESSAGE Machine kit (Ambion) and purified accordingly to the manufacturer's instructions. SIG-1R mRNAs were microinjected into 1- to 2-cell-stage embryos according to standard protocols. A volume of 1 nl was injected at a concentration of 100 ng/µl in each embryo.

***Western blot:*** Zebrafish larvae (n = 6; 5 days post fertilization, reared at 28 °C) were homogenized in 80 µl RIPA lysis buffer (50Mm Tris-HCI, pH 8, 150Mm NaCl, 0.5% sodium deoxycholate, 0.1% sodium dodecyl sulfate, and 1% Igepal CA-630) supplemented with cOmpleteTM protease inhibitor cocktail (Merck) and phenylmethylsulfonyl fluoride (Thermo fisher Scientific). Following a 1-min centrifugation, 1/5 (v/v final) sample Laemmli buffer was added to 65µl of supernatants. Total proteins were separated through a 10% polyacrylamide resolving gel, transferred to a nitrocellulose membrane (AmershamTM, Merck). Membrane was blocked for 1 h in the blocking solution (1X PBS, 0.1% Tween 20, and 5% dry milk) and incubated overnight with primary antibodies at 4 °C. Sig-1R protein was detected using a rabbit polyclonal antibody (1:100) generated by Abliance (Compiègne, France) and raised against residues 142-161(KSEVFYPGETVVHGPGEATAV) of human SIG-1R. Rabbit anti-Actin antibody (1/1600, Abcam) was used as a loading control. Secondary peroxidase-conjugated antibodies (1/5000, Jackson ImmunoResearch, Cambridge, UK) were incubated for 2 h in the blocking solution. Chemiluminescence was revealed by using the ClarityTM Western ECL Blotting substrates (Bio-Rad) and the ChemiDoc2 Touch Imaging System (Bio-Rad).

***Visual Motor Response (VMR):*** The VMR assay quantifies the locomotor activity of zebrafish larvae to light changes using infrared tracking system. At 4 days, individual larvae are transferred in wells of 96 well plate (Whatman #7701-1651 square and flat bottom wells) with 300 µl embryo medium and placed in the incubator at 28°C. *Wfs1ab^{WT}* larvae were deposited in even wells and *Wfs1ab^{KO}* in odd wells. Larvae that did not have swim bladders or presented visible physiological deformities were not used for the experiment. At 5-dpf, the locomotor behavior of larvae was monitored by using an automated videotracking device (Zebrabox^{®}, ViewPoint) by employing a DinionXF 1/3-inch Monochrome camera (model BFLY-PGE-13H2M, FLIR) fitted with a fixed-angle megapixel lens (SR5014MP-5C, SEIKO Optical). In the device, larvae in the plate are isolated from environmental surrounding noise. The floor of the box emitted by a light-controlling unit a white light (69-83 µW/cm2 measured at 495 λ). The response to stimuli was recorded by an infra-red (IR) camera (25 frames/s) under IR light illumination at 850 nm, which the animals could not perceive. The experiment started at 10:00 am and consisted in acclimating larvae to darkness (0% brightness) for 30 min, then switching light ON (100% brightness) for 10 min, then OFF (0% brightness) during 10 min. This was repeated twice, giving a total experiment duration of 70 min. The brightness changes were immediate (<< 1 s). The detection sensitivity was set to 31, activity burst threshold to 30 and activity freeze threshold to 10. Locomotor activity between the two threshold values was considered as normal activity. The distance covered was measured in mm/s. The values obtained in OFF were subtracted for each larvae by their ON values in order to remove the difference in basic locomotion that one could have between the groups.

***Drugs and administration procedures:*** PRE-084 was solubilised in zebrafish water and added to the dish containing zebrafish from 4 dpf to 5 dpf. The water containing the drug was changed the day of the experiment with fresh water containing the drug.

***Statistical analyses:*** Data were analyzed using two-way ANOVA with gender and genotype as independent factors, followed by a Newman-Keuls' *post-hoc* test. When a cut-off time was set, for passive avoidance latencies or water-maze swimming durations, data do not follow a Gaussian distribution and violin graphs showing median and interquartile range and non-parametric tests were used. Data were analyzed using Kruskal-Wallis ANOVA (H value) or a repeated-measures Friedman ANOVA (Q values), followed by a Dunn's *post-hoc* test. All ANOVA analyses are detailed in the supplementary material. One-column comparisons, *vs.* chance or zero levels (*i.e.,* 50% preference for object recognition or 15 s for the time spent in the pool quadrant) were performed using a one-column t-test. The level of statistical significance was *p* < 0.05. For reading clarity, all statistical data are detailed in supplementary materials.

### 2.2. Results

In WS patient fibroblasts, Wfs1 mutation resulted in a decreased expression of NCS-1 in MAMs, and thus in reduced NCS-1-induced modulation of IP₃R-gated Ca²⁺ transfer from the ER into the mitochondria. A significant 20% reduction in the expression level of NCS-1 is measured in the hippocampus and cortex of *Wfs1^{ΔExon8}* mice (Fig. 1a, 1b) and thus confirmed a direct repercussion of Wfs1 invalidation on NCS-1 levels. Among the numerous regulatory proteins modulating IP₃R activity, SIG-1R expression was not affected in the hippocampus (Fig. 1c) or cortex (Fig. 1d) of *Wfs1^{ΔExon8}* mice as compared with *Wfs1^{WT}* animals.

Male *Wfs1^{ΔExon8}* mice were administered with a selective SIG-1R agonist, PRE-084, at the most active dose *in vivo* and their learning abilities analyzed (Figs. 2-5). PRE-084-treated *Wfs1^{ΔExon8}* mice showed a very significant recovery of spontaneous alternation (Fig. 2). In the novel object test, the treatment failed to affect object exploration during session 2 (Fig. 3a) but attenuated the deficit of exploration for the novel object in session 3 (Fig. 3b). In the water-maze test, *Wfs1^{ΔExon8}* mice showed a delay in acquisition as compared to *Wfs1^{WT}* controls: first, significant differences in latencies were measured during swimming trials 3 to 5 (Fig. 4a). The daily injection of PRE-084 prevented this delay and significantly decreased swimming latencies during the two last trials (Fig. 4b). Calculation of the acquisition slopes confirmed that only vehicle (V)-treated *Wfs1^{ΔExon8}* mice showed a statistically significant decrease as compared to V-treated *Wfs1^{WT}* mice, but not PRE-084-treated *Wfs1^{ΔExon8}* mice (Fig. 4c). The probe test showed that all groups preferentially explored the T quadrant, confirming that *Wfs1^{ΔExon8}* mice finally acquired the spatial information, and no impact of the PRE-084 was noted (Fig. 4d). In the passive avoidance test, the step-through latency deficit as compared to V-treated *Wfs1^{WT}* mice was very significant for V-treated *Wfs1^{ΔExon8}* mice but not for PRE-084-treated *Wfs1^{ΔExon8}* mice (Fig. 5a). Moreover, the augmentation of escape latency measured in *Wfs1^{ΔExon8}* mice was significantly reduced after PRE-084 treatment (Fig. 5b).

Female *Wfs1^{ΔExon8}* mice were also treated with the PRE-084 SR1 agonist and examined in anxiety tests (Figs. 6-7). In the black-and-white exploration test, the PRE-084 treatment resulted in significant effects: the number of crossing was slightly reduced (Fig. 6a), the time spent in the light compartment and the time per visit were increased up to the levels of *Wfs1^{WT}* mice (Fig. 6b, 6c). In the elevated plus-maze, no impact was noted on maze exploration (Fig. 7a), but the time spent in open arms was slightly increased: the PRE-084-treated *Wfs1^{ΔExon8}* mice did not significantly differ from V-treated *Wfs1^{WT}* mice contrarily to V-treated *Wfs1^{ΔExon8}* mice (Fig. 7b). The time per visit in open arms was significantly increased after PRE-084 treatment in *Wfs1^{ΔExon8}* mice (Fig. 7c).

Interestingly, the availability of mutant zebrafish lines for the pathology could allow a direct confirmation of the pharmacological relevance on SIG-1R activity of an agonist treatment *vs.* a direct overexpression of the SIG-1R protein. Wfs1 gene is duplicated in zebrafish and the *Wfs1ab^{KO}* line, generated in the laboratory by crossing *Wfs1a^{KO}* and *Wfs1b^{KO}* mutants, was used. *Wfs1ab^{KO}* zebrafish showed a significant reduction in Wfsla mRNA levels, but only a tendency for Wfslb (Fig. 8a). mRNA levels of SIG-1R and NCS1, the latter also duplicated, were not affected in the mutant line (Fig. 8a). Protein levels for SIG-1R and NCS1 were checked and appeared unaffected (Fig. 8b). *Wfs1ab^{KO}* zebrafish showed a clear locomotor alteration when tested for their visual motor response (Fig. 9). Mobility during the light OFF periods was significantly increased in *Wfs1ab^{KO}* larvae. The PRE-084 treatment, tested between 0.1 and 10 µM in the bath, resulted in a concentration-dependent decrease of the hyperlocomotor response, down to the WT line mobility at 3 and 10 µM of the drug (Fig. 9). Moreover, we overexpressed SIG-1R by transitory transfection in the *Wfs1ab^{KO}* line and compared the mobility response with fish receiving only the mCherry control vector. SIG-1R overexpression in *Wfs1ab^{WT}* zebrafish induced a moderate but significant hypermobility response in *Wfs1ab^{WT}* fish, but it very significantly attenuated the hypermobility response observed in mCherry-treated *Wfs1ab^{KO}* larvae (Fig. 10a). Analysis of the KO/WT response level showed a +74% increase in mCherry-treated controls but only a +12% increase in SIG-1R overexpressing larvae (Fig. 10b).

Bibliographic references:
- Alon, A. et al., Proc Natl Acad Sci USA. 2017;114:7160-5. doi: 10.1073/pnas. 1705154114
- Anderson, T.R. et al., J Neurophysiol. 2005;93:963-79. doi: 10.1152/jn.00654.2004
- Goguadze, N. et al, Neurotox Res 2019;35:1-18.
- Gómez-Soler, M. et al., J Med Chem. 2014;57:238-42, doi: 10.1021/jm401529t
- Hayashi, T., Su, T.P. Cell. 2007;131:596-610. doi: 10.1016/j.cell.2007.08.036.
- Hayashi, T. et al., J Pharmacol Exp Ther 2000; 293:788-98.
- Luuk, H. et al., J Comp Neurol 2008;509:642-60.
- Maurice, T. Expert Opin Drug Discov. 2021 Apr;16(4):373-89.
- Maurice, T. et al., Brain Res. 1994a;647:44-56. doi: 10.1016/0006-8993(94)91397-8
- Maurice, T. et al., Pharmacol Biochem Behav. 1994b;49:859-69. doi: 10.1016/0091-3057(94)90235-6
- Minabe, Y. et al. Synapse. 1999;33:129-40. doi: 10.1002/(SICI)1098-2396(199908 33:2<129::AID-SYN3>3.0.CO;2-E)
- Monnet, F.P. et al., J Pharmacol Exp Ther. 1992;261:123-30.
- Monnet, F.P. et al., J Pharmacol Exp Ther. 1992;263:1219-25.
- Mori, T. et al., PLOS One. 2013;8:e76941. doi: 10.1371/journal.pone.0076941
- Vavers, E. et al., Front Pharmacol. 2019;10:223. doi: 10.3389/fphar.2019.00223
- Wang, L. et al., Mech Ageing Dev. 2012;133:665-74. doi: 10.1016/j.mad.2012.09.005
- Wu, Z., Bowen, B.D. J Biol Chem. 2008;283:28198-215.
- Yagasaki, Y. et al., J Biol Chem. 2006;281:12941-9. doi: 10.1074/jbc.M508157200
- Zhemkov, V. et al., eLife. 2021;10:e65192. doi: 10.7554/eLife.65192

## Claims

1. Compound for its use in the prevention or the treatment of a pathology associated with a mutation of WFS1, wherein said compound is an activator of Sigma-1 receptor (SIG-1R).

2. Compound according to claim 1 or 2, wherein said pathology is chosen among: Wolfram syndrome, Wolfram-like syndrome, *diabetes insipidus, diabetes mellitus,* optic atrophy, deafness, psychiatric disorders, acoustic trauma and severity of hearing loss following cisplatin treatment.

3. Compound according to any of the preceding claims, wherein said pathology is Wolfram syndrome.

4. Compound according to claim 1, wherein said SIG-1R activator is chosen among: i) SIG-1R agonists, ii) SIG-1R positive modulators and iii) Sig-1R or a fragment thereof.

5. Compound according to any of the preceding claims, wherein said compound is chosen among the groups of: molecules, peptides and steroids.

6. Compound according to any of the preceding claims, wherein said compound is 2-(4-morpholinethyl)-1-phenylcyclohexanecarboxylate hydrochloride (PRE-084).

7. Pharmaceutical composition comprising, as an active ingredient, an activator of SIG-1R or a pharmacologically acceptable salt thereof, for its use in the prevention or the treatment of a pathology associated with a mutation of WFS1.

8. Pharmaceutical composition according to claim 7, wherein said pathology is Wolfram syndrome.

9. Pharmaceutical composition according to claim 7 or 8, wherein said SIG-1R activator is a SIG-1R agonist.
